Europäisches Patentamt

European Patent Office

Office européen des brevets

(11) Veröffentlichungsnummer: **0 313 910 B1**

(12) **EUROPÄISCHE PATENTSCHRIFT**

(45) Veröffentlichungstag der Patentschrift: **15.01.92**

(21) Anmeldenummer: **88116822.3**

(22) Anmeldetag: **11.10.88**

(51) Int. Cl.⁵: **C07C 45/78**, C07C 45/81, C07C 45/34, C07C 47/06

(54) **Verfahren zur Abtrennung organischer Chlorverbindungen aus dem Abgas der Etylenoxidation.**

(30) Priorität: **28.10.87 DE 3736459**

(43) Veröffentlichungstag der Anmeldung:
**03.05.89 Patentblatt 89/18**

(45) Bekanntmachung des Hinweises auf die Patenterteilung:
**15.01.92 Patentblatt 92/03**

(84) Benannte Vertragsstaaten:
**CH DE ES IT LI**

(56) Entgegenhaltungen:
**EP-A- 0 006 523**
**DE-A- 3 610 706**

**PATENT ABSTRACTS OF JAPAN, Band 6, Nr. 35 (C-93)[913], 3. März 1982; & JP-A-56 152 433 (MITSUBISHI KASEI KOGYO K.K.) 26-11-1981**

(73) Patentinhaber: **HOECHST AKTIENGESELL-SCHAFT**
**Postfach 80 03 20**
**W-6230 Frankfurt am Main 80(DE)**

(72) Erfinder: **Erpenbach, Heinz, Dr.**
**Oberbuschweg 22**
**W-5000 Köln(DE)**
Erfinder: **Gehrman, Klaus, Dr.**
**Geschwister-Scholl-Strasse 32**
**W-5042 Erftstadt(DE)**
Erfinder: **Dahmen, Hubert, Dr.**
**Nibelungenstrasse 9**
**W-5030 Hürth(DE)**
Erfinder: **Lork, Winfried**
**Siegfried-von-Westenburg-Strasse 14**
**W-5042 Erftstadt(DE)**

## Beschreibung

Die Erfindung betrifft ein Verfahren zur Abtrennung organischer Chlorverbindungen aus dem Abgas, das bei der Umsetzung von Ethylen mit Sauerstoff zu Acetaldehyd in Gegenwart eines aus Palladium und Kupfer oder ihrer Verbindungen in salzsaurer wäßriger Lösung bestehenden Katalysatorsystems anfällt (K. Weissermel/H.J. Arpe, Industrielle org. Chemie, Verlag Chemie, 1976, Seiten 137-139.

Die Oxidationsprodukte des Ethylens werden gemeinsam mit den gasförmigen Produkten aus dem Reaktor abgezogen. Nach Isolierung des Acetaldehyds durch eine Wasserwäsche wird der überwiegend aus Ethylen bestehende Gasstrom im Kreislauf in die Reaktion zurückgeführt. Zur Vermeidung einer Anreicherung unerwünschter Nebenprodukte wie $CO_2$, Methan, Ethan und organische Chlorverbindungen sowie von inerten Gasen wie Stickstoff und Argon im Kreislaufgas wird ein Teilstrom als Abgas ausgeschleust. Dieser Abgasstrom enthält die organischen Chlorverbindungen, die entsprechend ihrem Partialdruck in der Gasphase vorliegen. Die organischen Chlorverbindungen des abgezogenen Gasstromes wirken sich auf die weitere Verwendung der darin enthaltenen Bestandteile, insbesondere des Ethylens, nachteilig aus. Eine Abtrennung der organischen Chlorverbindungen aus dem Abgasstrom ist deshalb unerläßlich, damit das enthaltene Ethylen Folgereaktionen zugeführt und zum andern ein gereinigtes Abgas über eine Fackel in die Umwelt abgeführt werden kann. Im Gasstrom liegen die organischen Chlorverbindungen überwiegend als Methylchlorid neben Ethylchlorid vor.

Die vorliegende Erfindung erlaubt eine nahezu quantitative Abtrennung der organischen Chlorverbindungen aus den bei der Ethylenoxidation zu Acetaldehyd anfallenden Abgasen durch Gegenstromextraktion mit polaren organischen Lösemitteln.

Das Verfahren der Erfindung ist nun dadurch gekennzeichnet, daß man das nach Isolierung der Reaktionsprodukte ausgeschleuste Abgas bei Temperaturen von 233 K bis 323 K und Drücken von 1-10 bar im Gegenstrom mit polaren organischen Lösemitteln auswäscht.

Weiterhin kann das Verfahren der Erfindung bevorzugt und wahlweise dadurch gekennzeichnet sein, daß

a) man als polare organische Lösemittel N-Methylpyrrolidon, Dimethylsulfoxid, Dimethylformamid, Diethylenglykoldimethylether oder Triethylenglykoldimethylether verwendet;

b) das Volumenverhältnis von Abgas zu Lösemittel in der Gegenstromwäsche (10 bis 300) : 1, vorzugsweise (50 bis 200) : 1, beträgt;

c) man die Gegenstromwäsche bei Temperaturen von 253 K bis 303 K und Drücken von 1-5 bar durchführt;

d) das Abgas Ethylen, Kohlendioxid, Sauerstoff, Argon, Stickstoff, Methan, Ethan sowie mindestens einer organischer Chlorverbindung aus der Gruppe Methylchlorid und Ethylchlorid enthält;

e) man das im Ablauf der Gegenstromwäsche mit organischen Chlorverbindungen beladene Lösemittel einer Trennstufe zuführt, die organischen Chlorverbindungen dampfförmig abtrennt und das so von organischen Chlorverbindungen befreite Lösemittel erneut in die Gegenstromwäsche einsetzt;

f) man das Lösemittel durch Abstreifen mit Stickstoff von den organischen Chlorverbindungen befreit.

Durch das Verfahren der Erfindung werden die organischen Chlorverbindungen nahezu quantitativ aus dem Abgasstrom entfernt. Der gereinigte Abgasstrom enthält überwiegend Ethylen neben Kohlendioxid, Methan und Ethan, Inertgasen und Sauerstoff. Er kann für Folgereaktionen eingesetzt werden.

Die durch Extraktion aus dem Abgas entfernten organischen Chlorverbindungen werden in einer Trennstufe gemeinsam mit gelösten Anteilen des Abgases vom Lösemittel abgetrennt. Sie lassen sich durch Destillation reinigen oder können gegebenenfalls in einer Verbrennungsanlage als Brennstoff dienen.

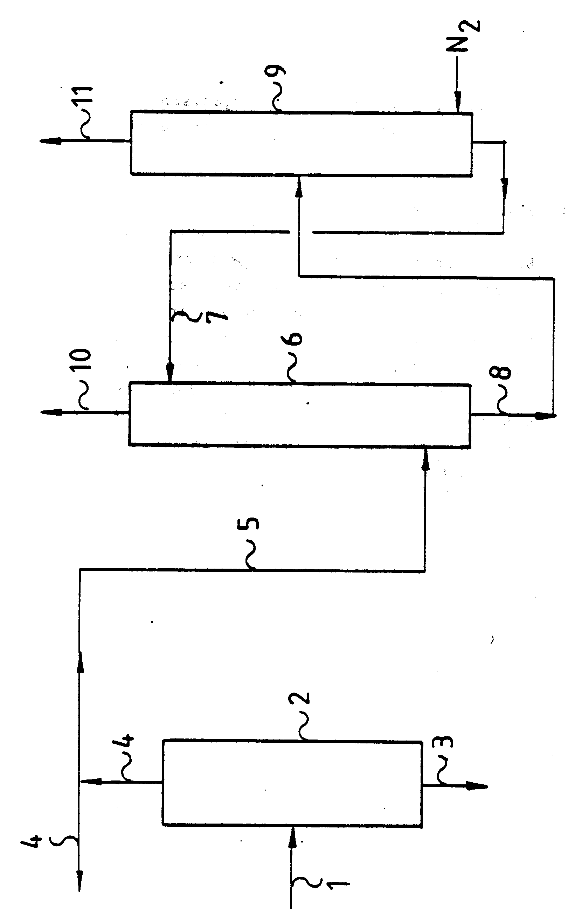

Die Erfindung wird im folgenden anhand der Zeichnung näher erläutert:

Aus dem Reaktor wird das bei der Oxidation von Ethylen mit Sauerstoff in Gegenwart eines aus Palladium und Kupfer bzw. ihrer Verbindungen in salzsaurer wäßriger Lösung bestehenden Katalysatorsystems erhaltene Reaktionsprodukt gemeinsam mit dem Kreislaufgas abgezogen und über Leitung 1 einer Waschkolonne 2 zugeführt. Die Waschkolonne 2 wird auf einem Druck von 1-5 bar gehalten. Der durch die Leitung 1 zugeführte Produktstrom wird in der Waschkolonne 2 mit Wasser im Gegenstrom gewaschen und in einen flüssigen Anteil, der über Leitung 3 abgezogen wird, und in einen gasförmigen Anteil aufgetrennt. Der am Kopf der Waschkolonne 2 vorzugsweise mit einer Temperatur von 288-303 K ankommende Gasstrom wird über Leitung 4 abgezogen und im Kreislauf zum Reaktor zurückgeführt. Ein Teilstrom wird als Abgas ausgeschleust und über Leitung 5 dem unteren Boden der Waschkolonne 6 zugeführt, die zur Erhöhung des Kontaktes zwischen Gas und Flüssigkeit Glockenböden oder Füllkörper enthält. Das Abgas enthält neben den Reaktionsprodukten Kohlendioxid, Methan, Ethan und organischen Chlorverbindungen überwiegend nichtumgesetztes Ethylen sowie Inertgase (Stickstoff, Argon). Am Kopf der Waschkolonne 6 wird über Leitung 7 das polare organische Lösemittel mit einer Temperatur von 233-323 K aufgegeben und das nach oben strömende Abgas im Gegenstrom gewaschen. Das Verhältnis der Volumina Gas zu Waschflüssigkeit (polares organisches Lösemittel) beträgt 10-300 : 1, vorzugsweise 50-200 : 1. Die mit organischen Chlorverbindungen beladene Waschflüssigkeit wird über Leitung 8 abgezogen und zur Trennstufe 9 geführt. Das gereinigte Abgas kann über Leitung 10 entnommen werden und für Folgereaktionen Verwendung finden. In der Trennstufe 9 werden die organischen Chlorverbindungen sowie die gelösten Anteile des Abgases aus dem polaren organischen Lösemittel über Leitung 11 entfernt. Sie können in einer weiteren Trennstufe gereinigt werden oder ggf. in einer Verbrennungsanlage als Brennstoffe Verwendung finden. Das Lösemittel wird anschließend über Leitung 7 der Waschkolonne 6 erneut aufgegeben.

In den nachfolgenden Beispielen beziehen sich die Literangaben auf Normalbedingungen (273, 16 K und 1,013 bar).

Beispiel 1

Zur Aufrechterhaltung eines konstanten Gaskreislaufs wird aus dem Reaktionsgas der Ethylenoxidation nach Gewinnung der Flüssigprodukte ein Abgasstrom mit der Zusammensetzung: 77,2 Vol.% Ethylen, 0.5 Vol.% Methan und Ethan, 10,5 Vol% Kohlendioxid, 7,3 Vol.% Stickstoff und Argon, 4,2 Vol.% Sauerstoff sowie 0,3 Vol.% Methylchlorid entnommen. Zur Entfernung des Methylchlorids werden 300 l/h dieses Abgases aus Leitung 5 bei einer Temperatur von 298 K und einem Druck von 4 bar am Boden einer mit 4 mm Berlsattelkörpern gefüllten Waschkolonne 6 (Füllhöhe: 2 m, Durchmesser: 25 mm) eingeleitet und mit 1,5 l/h N-Methylpyrrolidon, welches oberhalb der Füllkörperschicht über Leitung 7 eingespeist wird, im Gegenstrom in Berührung gebracht. Am Kopf der Waschkolonne 6 fallen stündlich 289 l gereinigtes Abgas in der Zusammensetzung: 77,4 Vol.% Ethylen, 0,5 Vol.% Methan und Ethan, 10,6 Vol.% Kohlendioxid, 7,3 Vol.% Stickstoff und Argon, 4,2 Vol.% Sauerstoff und < 0,01 Vol.% Methylchlorid an. Das am Boden abfließende und mit Methylchlorid sowie weiteren Abgasbestandteilen belandene N-Methylpyrrolidon gelangt nach dem Entspannen über Leitung 8 in die Trennstufe 9 (Desorptionskolonne; Höhe der 6-mm-Berlsattelkörper-Schicht: 1,5 m, Durchmesser: 25 mm). Unter einem Druck von 1 bar wird das oberhalb der Füllkörperschicht eindosierte N-Methylpyrrolidon zur Desorption der gasförmigen Bestandteile mit 2 l/h in den Sumpf der Trennstufe 9 eingeleiteten Stickstoffs bei einer Temperatur von 475 K gestrippt. Am Kondensator des Kolonnenkopfes werden dabei stündlich 13 l Gas mit der Zusammensetzung: 6,9 Vol.% Methylchlorid, 60,0 Vol.% Ethylen, 0,5 Vol.% Ethan und Methan, 8,5 Vol.% Kohlendioxid, 21,0 Vol.% Stickstoff und Argon sowie 3,1 Vol.% Sauerstoff abgesogen. Sie werden einer Verbrennungsanlage zugeführt oder zur Gewinnung von Methylchlorid weiter aufgearbeitet. Durch die Extraktion gelingt eine Anreicherung des $CH_3Cl$ im Abgas um den Faktor 23. Das am Boden der Trennstufe 9 abfließende N-Methylpyrrolidon enthält keine nachweisbaren gasförmigen Bestandteile. Nach Kühlung wird es wieder auf den Kopf der Waschkolonne 6 zurückgeführt.

Beispiel 2

Zur Aufrechterhaltung eines konstanten Gaskreislaufs wird aus dem Reaktionsgas der Ethylenoxidation nach Gewinnung der Flüssigprodukte ein Abgasstrom mit der Zusammensetzung: 78,5 Vol.% Ethylen, 9,7 Vol.% Kohlendioxid, 7,3 Vol.% Stickstoff und Argon, 3,8 Vol.% Sauerstoff sowie 0,7 Vol.% Methylchlorid entnommen. Zur Entfernung des Methylchlorids werden 200 l/h dieses Abgases aus Leitung 5 bei einer Temperatur von 253 K und einem Druck von 1 bar am Boden einer mit 22 Glockenböden bestückten Waschkolonne 6 (Durchmesser: 50 mm) eingeleitet und mit 2 l/h N-Methylpyrrolidon, welches am obersten Boden der Kolonne über Leitung 7 eingespeist wird, im Gegenstrom in Berührung gebracht. Am Kopf der Waschkolonne 6 werden stündlich 192 l gereinigtes Abgas mit der Zusammensetzung: 79,1 Vol.% Ethylen, 9,7 Vol.% Kohlendioxid, 7,3 Vol.% Stickstoff und Argon, 3,8 Vol.% Sauerstoff und < 0,01 Vol.% Methylchlorid erhalten. Das am Boden abfließende und mit Methylchlorid sowie weiteren Abgasbestandteilen beladene N-Methylpyrrolidon wird über Leitung 8 in die Trennstufe 9 (Desorptionskolonne) geführt. Bei einer Temperatur von 475 und einem Druck von 1 bar wird das oberhalb der Füllkörperschicht eindosierte N-Methylpyrrolidon zur Desorption der gasförmigen Bestandteile mit 0,5 l/h in den Sumpf der Trennstufe 9 eingeleiteten Stickstoffs gestrippt. Über den Kondensator der Kolonne werden dabei stündlich 8,5 l Gas mit der Zusammensetzung: 16,5 Vol.% Methylchlorid, 61,2 Vol.% Ethylen, 8,2 Vol.% Kohlendioxid, 11,8 Vol.% Stickstoff und Argon sowie 2,3 Vol.% Sauerstoff abgezogen. Sie gelangen in eine Verbrennungsanlage oder werden zur Gewinnung von Methylchlorid weiter aufgearbeitet. Unter diesen Bedingungen wird durch die Extraktion eine Anreicherung des Methylchlorids im Abgas um den Faktor 23,6 erreicht.

Das am Boden der Trennstufe 9 abfließende N-Methylpyrrolidon enthält keine nachweisbaren gasförmigen Bestandteile Nach Kühlung wird es erneut zur Extraktion eingesetzt.

Beispiel 3

Zur Aufrechterhaltung eines konstanten Gaskreislaufs wird aus dem Reaktionsgas der Ethylenoxidation nach Gewinnung der Flüssigprodukte ein Abgasstrom mit der Zusammensetzung: 76,1 Vol.% Ethylen, 10,8 Vol.% Kohlendioxid, 8,4 Vol.% Stickstoff und Argon, 3,5 Vol.% Sauerstoff sowie 1,1 Vol.% Methylchlorid und 0,1 Vol.% Ethylchlorid entnommen. zur Entfernung des Methylchlorids werden 200 l/h dieses Abgases aus Leitung 5 bei einer Temperatur von 273 K und einem Druck von 2 bar am Boden einer mit 22 Glockenböden bestückten Waschkolonne 6 eingeleitet und mit 3 l/h Triethylenglykoldimethylether (Triglyme), welches am obersten Boden der Kolonne über Leitung 7 eingespeist wird, im Gegenstrom in Berührung gebracht. Am Kopf der Waschkolonne 6 fallen stündlich 190,5 l gereinigtes Abgas mit der Zusammensetzung: 77,0 Vol.% Ethylen, 10,9 Vol.% Kohlendioxid, 8,5 Vol.% Stickstoff und Argon, 3,6 Vol.% Sauerstoff sowie 0,01 Vol.% Methylchlorid und 0,01 Vol.% Ethylchlorid an. Das am Boden abfließende und mit Methylchlorid, Ethylchlorid sowie weiteren Abgasbestandteilen beladene Triglyme gelangt nach dem Entspannen über Leitung 8 in die Trennstufe 9 (Desorptionskolonne).
Bei einer Temperatur von 495 K und einem Druck von 1 bar wird das oberhalb der Füllkörperschicht eindosierte Triglyme zur Desorption der gasförmigen Bestandteile mit 1 l/h in den Sumpf der Trennstufe 9 eingeleiteten Stickstoffs gestrippt. Am Kondensator des Kolonnenkopfes werden dabei stündlich 10,5 l Gas mit der Zusammensetzung: 21,0 Vol.% Methylchlorid, 1,9 Vol.% Ethylchlorid, 51,4 Vol.% Ethylen, 8,6 Vol.% Kohlendioxid, 15,2 Vol.% Stickstoff und Argon sowie 1,9 Vol.% Sauerstoff abgezogen. Sie werden entweder zur Dampferzeugung verbrannt oder zur Gewinnung von Methylchlorid weiter aufgearbeitet. Das am Boden der Trennstufe 9 abfließende Triglyme enthält keine nachweisbaren gasförmigen Bestandteile. Nach Kühlung wird es im Kreislauf der Waschkolonne 6 wieder aufgegeben.

Beispiel 4

Zur Aufrechterhaltung eines konstanten Gaskreislaufs wird aus dem Reaktionsgas der Ethylenoxidation nach Gewinnung der Flüssigprodukte ein Abgasstrom mit der Zusammensetzung: 77,7 Vol.% Ethylen, 10,5 Vol.% Kohlendioxid, 7,3 Vol.% Stickstoff und Argon, 4,2 Vol.% Sauerstoff sowie 0,3 Vol.% Methylchlorid entnommen. Zur Entfernung des Methylchlorids werden 3500 l/h dieses Abgases aus Leitung 5 bei einer Temperatur von 298 K und einem Druck von 4 bar am Boden einer mit 6 mm Berlsattelkörpern gefüllten Waschkolonne 6 (Füllhöhe 2,5 m, Durchmesser 35 mm) eingeleitet und mit 20 l/h Dimethylformamid, welches oberhalb der Füllkörperschicht über Leitung 7 eingespeist wird, im Gegenstrom in Berührung gebracht. Am Kopf der Waschkolonne 6 fallen stündlich 3290 l gereinigtes Abgas mit der Zusammensetzung: 78,0 Vol.% Ethylen, 10,5 Vol.% Kohlendioxid, 7,3 Vol.% Stickstoff und Argon, 4,2 Vol.% Sauerstoff und < 0,01 Vol.% Methylchlorid an. Das am Boden abfließende und mit Methylchlorid sowie weiteren Abgasbestandteilen beladene Dimethylformamid gelangt über Leitung 8 in die Trennstufe 9 (Desorptionskolonne). Unter einem Druck von 1,8 bar wird das oberhalb der Füllkörperschicht eindosierte Dimethylformamid zur Desorption der gasförmigen Bestandteile im Verdampfer der Trennstufe 9 auf eine Temperatur von 450 K aufgeheizt. Am Kondensator des Kolonnenkopfes werden dabei stündlich 210 l Gas mit der Zusammensetzung: 5,0 Vol.% Methylchlorid, 73,7 Vol.% Ethylen, 10,0 Vol.% Kohlendioxid, 7,1 Vol.% Stickstoff und Argon sowie 4,2 Vol.% Sauerstoff abgezogen. Sie werden entweder zur Dampferzeugung verbrannt oder zur Gewinnung von Methylchlorid weiter aufgearbeitet.

Das am Boden der Trennstufe 9 abfließende Dimethylformamid enthält keine nachweisbaren gasförmigen Bestandteile. Nach Kühlung wird es im Kreislauf der Waschkolonne 6 wieder aufgegeben.

Beispiel 5

Zur Aufrechterhaltung eines konstanten Gaskreislaufs wird aus dem Reaktionsgas der Ethylenoxidation nach Gewinnung der Flüssigprodukte ein Abgasstrom mit der Zusammensetzung: 77,8 Vol.% Ethylen, 10,1 Vol.% Kohlendioxid, 7,8 Vol.% Stickstoff und Argon, 4,0 Vol.% Sauerstoff sowie 0,3 Vol.% Methylchorid entnommen. Zur Entfernung des Methylchlorids werden 2500 l/h dieses Abgases aus Leitung 5 bei einer Temperatur von 298 K und einem Druck von 4 bar am Boden einer mit 11 Glockenböden bestückten Waschkolonne 6 (Durchmesser 25 mm) eingeleitet und mit 20 l/h Dimethylsulfoxid, welches über Leitung 7 eingespeist wird, im Gegenstrom in Berührung gebracht. Am Kopf der Waschkolonne 6 fallen stündlich 2390 l gereinigtes Abgas mit der Zusammensetzung: 78,3 Vol.% Ethylen, 9,6 Vol.% Kohlendioxid, 8,0 Vol.% Stickstoff und Argon, 4,1 Vol.% Sauerstoff und < 0,01 Vol.% Methylchlorid an. Das am Boden abfließende und mit Methylchlorid sowie weiteren Abgasbestandteilen beladene Dimethylsulfoxid gelangt über Leitung 8 in die Trennstufe 9 (Desorptionskolonne). Unter einem Druck von 1,8 bar wird das oberhalb der Füllkörperschicht eindosierte Dimethylsulfoxid zur Desorption der gasförmigen Bestandteile im Verdampfer der Trennstufe 9 auf eine Temperatur von 483 K aufgeheizt. Am Kondensator des Kolonnenkopfes werden dabei stündlich 110 l Gas mit der Zusammensetzung: 6,8 Vol.% Methylchlorid, 65,6 Vol.% Ethylen, 21,6 Vol.% Kohlendioxid, 4,1 Vol.% Stickstoff und Argon sowie 1,9 Vol.% Sauerstoff abgezogen. Sie werden entweder zur Dampferzeugung verbrannt oder zur Gewinnung von Methylchlorid weiter aufgearbeitet.

Das am Boden der Trennstufe 9 abfließende Dimethylsulfoxid enthält keine nachweisbaren gasförmigen Bestandteile. Nach Kühlung wird es im Kreislauf der Waschkolonne 6 wieder aufgegeben.

**Patentansprüche**

1. Verfahren zur Abtrennung organischer Chlorverbindungen aus dem Abgas, das bei der Umsetzung von Ethylen mit Sauerstoff zu Acetaldehyd in Gegenwart eines aus Palladium und Kupfer oder ihrer Verbindungen in salzsaurer wäßriger Lösung bestehenden Katalysatorsystems anfällt, dadurch gekennzeichnet, daß man das nach Isolierung der Reaktionsprodukte ausgeschleuste Abgas bei Temperaturen von 233 K bis 323 K und Drücken von 1-10 bar im Gegenstrom mit polaren organischen Lösemitteln auswäscht.

2. Verfahren nach Anspruch 1, dadurch gekennzeichnet, daß man als Lösemittel N-Methylpyrrolidon, Dimethylsulfoxid, Dimethylformamid, Diethylenglykoldimethylether oder Triethylenglykoldimethylether verwendet.

3. Verfahren nach Anspruch 1 oder 2, dadurch gekennzeichnet, daß das Volumenverhältnis von Abgas zu Lösemittel in der Gegenstromwäsche (10 bis 300) : 1, vorzugsweise (50 bis 200) : 1, beträgt.

4. Verfahren nach einem der vorhergehenden Ansprüche, dadurch gekennzeichnet, daß man die Gegenstromwäsche bei Temperaturen von 253 K bis 303 K und Drücken von 1-5 bar durchführt.

5. Verfahren nach einem der vorhergehenden Ansprüche, dadurch gekennzeichnet, daß das Abgas Ethylen, Kohlendioxid, Sauerstoff, Argon, Stickstoff, Methan, Ethan sowie mindestens eine organische Chlorverbindung aus der Gruppe Methylchlorid und Ethylchlorid enthält.

6. Verfahren nach einem der vorhergehenden Ansprüche, dadurch gekennzeichnet, daß man das im Ablauf der Gegenstromwäsche mit organischen Chlorverbindungen beladene Lösemittel einer Trennstufe zuführt, die organischen Chlorverbindungen dampfförmig abtrennt und das so von organischen Chlorverbindungen befreite Lösemittel erneut in die Gegenstromwäsche einsetzt.

7. Verfahren nach Anspruch 6, dadurch gekennzeichnet, daß man das Lösemittel durch Abstreifen mit Stickstoff von den organischen Chlorverbindungen befreit.

**Claims**

1. A process for removing organochlorine compounds from the offgas produced on reaction of ethylene with oxygen to form acetaldehyde in the presence of a catalyst system comprising palladium and copper or compounds thereof in aqueous hydrochloric acid solution, which comprises washing the offgas channeled off after isolation of the reaction products, at temperatures from 233 K to 323 K and pressures from 1-10 bar in countercurrent with polar organic solvents.

2. A process as claimed in claim 1, wherein the solvent used is N-methylpyrrolidone, dimethyl sulfoxide, dimethylformamide, diethylene glycol dimethyl ether or triethylene glycol dimethyl ether.

3. A process as claimed in claim 1 or 2, wherein the volume ratio between the offgas and the solvent in the countercurrent wash is (10 to 300) : 1, preferably (50 to 200) : 1.

4. A process as claimed in any of the preceding claims, wherein the countercurrent wash is carried out at temperatures from 253 K to 303 K and at pressures from 1-5 bar.

5. A process as claimed in any of the preceding claims, wherein the offgas contains ethylene, carbon dioxide, oxygen, argon, nitrogen, methane, ethane and at least one organochlorine compound from the group comprising methyl chloride and ethyl chloride.

6. A process as claimed in any of the preceding claims, wherein the solvent charged with organochlorine compounds during the countercurrent wash is fed to a separation step, the organochlorine compounds are removed in vapor form, and the solvent freed from organochlorine compounds in this manner is re-employed in the countercurrent wash.

7. A process as claimed in claim 6, wherein the solvent is freed from the organochlorine compounds by stripping with nitrogen.

**Revendications**

1. Procédé pour la séparation de composés chlorés organiques d'un gaz résiduaire qui se forme dans la réaction de l'éthylène avec l'oxygène en acétaldéhyde en présence d'un système catalyseur consistant en palladium et cuivre ou leurs composés en solution aqueuse chlorhydrique, caractérisé en ce que l'on lave le gaz résiduaire séparé après isolement des produits de réaction à contre-courant avec des solvants organiques polaires à des températures de 233 K à 323 K et sous des pressions de 1-10 bar.

2. Procédé selon la revendication 1, caractérisé en ce que l'on utilise comme solvant la N-méthylpyrrolidone, le diméthylsulfoxyde, le diméthylformamide, l'éther diméthylique du diéthylèneglycol ou l'éther diméthylique du triéthylèneglycol.

3. Procédé selon la revendication 1 ou 2, caractérisé en ce que le rapport en volume du gaz résiduaire au solvant dans le lavage à contre-courant est de (10 à 300) : 1, de préférence de (50 à 200) : 1.

4. Procédé selon l'une des revendications précédentes, caractérisé en ce que l'on met en oeuvre le lavage à contre-courant à des températures de 253 K à 303 K et sous des pressions de 1-5 bar.

5. Procédé selon l'une des revendications précédentes, caractérisé en ce que le gaz résiduaire contient de l'éthylène, du dioxyde de carbone, de l'oxygène, de l'argon, de l'azote, du méthane, de l'éthane et au moins un composé chloré organique du groupe du chlorure de méthyle et du chlorure d'éthyle.

6. Procédé selon l'une des revendications précédentes, caractérisé en ce que l'on envoie le solvant chargé en composés chlorés organiques au cours du lavage à contre-courant à une étape de séparation qui sépare les composés chlorés organiques à l'état vapeur et on utilise à nouveau dans le lavage à contre-courant le solvant ainsi débarrassé des composés chlorés organiques.

7. Procédé selon la revendication 6, caractérisé en ce que l'on débarrasse le solvant des composés chlorés organiques par entraînement par l'azote.

EP 0 313 910 B1